# EUROPEAN PATENT SPECIFICATION

(11) **EP 3 270 720 B1**
(45) Date of publication and mention of the grant of the patent: **02.01.2019**
(21) Application number: 16719470.3
(22) Date of filing: 17.03.2016
(51) Int. Cl.: A41B 11/00, A61F 13/08

(54) **DIFFERENTIATED LYMPH DRAINAGE GARMENT**
DIFFERENZIERTES LYMPHDRAINAGEKLEIDUNGSSTÜCK
VÊTEMENT DE DRAINAGE LYMPHATIQUE DIFFÉRENCIÉ

(30) Priority: 20.03.2015 IT BA20150025
(43) Date of publication of application: 24.01.2018
(73) Proprietor: Defente, Ruggiero, 76121 Barletta (BT) (IT); Paparella, Salvatore, 76121 Barletta (BT) (IT)
(72) Inventor: Defente, Ruggiero, 76121 Barletta (BT) (IT); Paparella, Salvatore, 76121 Barletta (BT) (IT)
(74) Representative: Russo, Dimitri
(86) International application number: PCT/IB2016/000311
(87) International publication number: WO 2016/151383

(56) References cited:
- WO-A1-2014/188250
- US-A1- 2014 082 815
- US-B1- 6 430 752

## Description

The invention relates to the field of medical devices for the treatment of diseases related to blood circulation and, more specifically, the object of the present invention are pants capable of improving the lymph drainage of adipose tissues of the legs.

As is known, the term lymphedema defines an anomaly of the lymphatic system which causes an abnormal accumulation of lymph especially in the limbs.

The triggering factors of this condition of accumulation of lymph can be, in addition to lifestyle, too tight clothing which excessively compress the limbs, obstructing both the blood and the lymphatic circulation, giving rise to the so-called orange peel skin and imperfections such as fat deposits on thighs, hips and buttocks.

A poor hemolymph circulation causes a poor blood and lymph inflow to the tissues, resulting in poor nutrition of the same, poor oxygen supply and reduced ability to eliminate toxins, resulting in the accumulation of harmful substances.

Techniques for improving or at least maintaining the health of the blood and lymphatic circulation are known. Aside from the surgical techniques used for advanced clinical cases, as well as the pharmacological methodologies, the currently most used techniques are those related to lymphatic drainage massage and to the elastic compression technology; in particular, the latter involve the use of elastic stockings or bands.

Bands, definitely unsightly and unpleasant to patients, are very useful and are used for more serious diseases, thus becoming irreplaceable.

However, their use is mostly temporary, to allow healing of an injury (immediate post-operative, after sclerotherapy, superficial thrombophlebitis), or to return the system back to balance (edema, post-thrombotic syndrome, ulcers).

They are also used to eliminate the swelling (edema) present in the legs as a result of venous hypertension.

They are also useful in the treatment of ulcers, especially in the early stage.

However, said bands have a number of drawbacks and, in particular, in addition to the blemishes listed above, they are difficult to apply by the nurses and by the patient or his/her relatives.

In contrast, stockings are better appreciated by doctors and patients as they are aesthetically nicer and apparently easier to wear. However, they have the drawback of being less effective than the bands and have a higher cost. Moreover, heavier stockings are difficult to wear, especially for older people.

Pants are also known which are made with a fabric divided into bands with different elasticity, decreasing from the bottom upwards. WO2014/188250 discloses a pair of lymph drainage trousers characterised by a series of bands having decreasing elasticity.

The object of the present invention is to provide a device which provides a lymphatic drainage massage to improve the blood and lymphatic circulation by means of pants which provide a decreasing graduated compression starting from the ankles.

A further object is to provide a garment which solves the problems related to the inherent blemishes of known devices and which is particularly suitable for improving the blood and lymphatic circulation, helping to improve aesthetic imperfections such as cellulite and the so-called orange peel skin.

Yet another object is to provide a piece of clothing with elasticity decreasing from the top downwards but differentiated between the front and back part. The difference between the two panels allows preserving the graduated compression, respecting the anatomical front-back differences, enhancing the fit and the modeling effect and comfort, as well as creating in the band compression, thanks to the difference between the front and back bands, a further intermediate compression band (since it is the average of the two bands) which softens the pressure change between one band and the other.

Yet another object is to provide pants with elastic bands the fabric of which comprises mineral components in order to obtain a garment capable of producing heat when wet by perspiration or by the physiological hydrolipid film, and retain it and release it slowly.

Another object is to provide a garment the fabric of which comprises mineral components capable of releasing infrared rays, with positive effects on the vascular health in general.

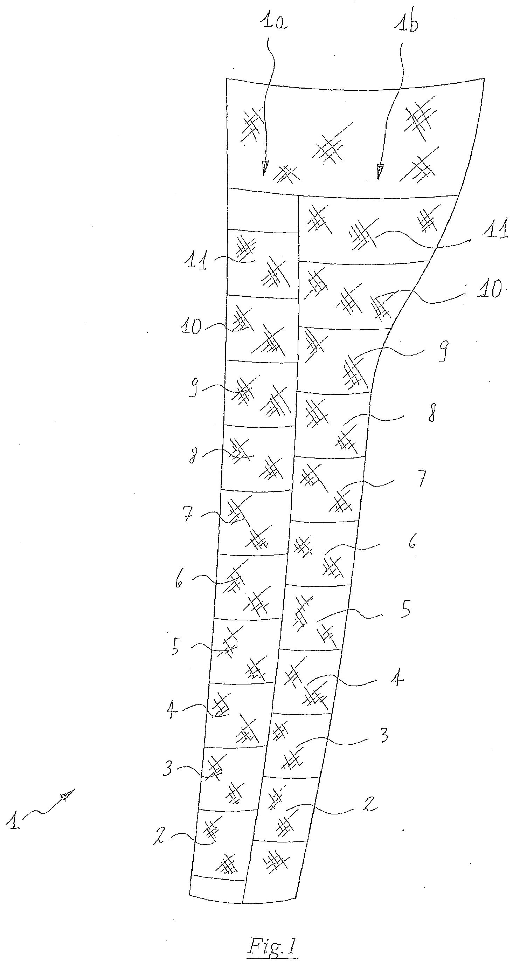

The differentiated lymph drainage garment according to the present invention is described hereinafter, in a preferred non-limiting embodiment of further developments in the scope of the invention itself, with the aid of the accompanying drawing which illustrates the following figure: Fig. 1, a side view of a leg of pants made according to the present invention, wherein the bands with upwards decreasing concentration of the elastomers are highlighted.

According to the present invention, garment 1 uses a fabric formed by areas whose concentration of elastomers gradually varies.

More in particular, as shown in the accompanying figure, the garment which for example is pants, comprises a front part 1a and a back part 1b, each comprising a plurality of bands from 2 to 11 whose elasticity, i.e. the degree of compression capable of exerting on the user's body, does not coincide but, in both cases, decreases from the bottom upwards.

The progressive elasticity is achieved by inserting a greater number of elastomers in the weft.

The maximum area of pressure, about 20mmhg, is made at the front of the ankle, i.e. in the fabric band which has the highest concentration of elastomers and it progressively decreases in the bands close to the crotch of the pants, for example at knee height, the pressure exerted by band 6 is about 10 mmHg, in contrast band 11 in the proximity of the waist will exert a pressure of about 1mmhg.

The slow graduated, decreasing and constant pressure only partially coinciding at the front with respect to the back, supports and facilitates the lymphatic circulation, ensuring a stimulus of the same, preventing the lymphatic stasis which leads to the problems already mentioned.

The lymph drainage pants 1, through the walking of its user, create a pumping with an effect on the lymphatic vessels which contract and move up the lymph, draining the connective tissue. The beneficial effects of the garment are also amplified by the fact that the fabric comprises zeolite and steatite components.

According to the present invention, garment 1 is made according to the following steps:
1. Tailoring a garment comprising a front fabric piece 1a, formed by a plurality of bands with different elasticity, and a back fabric piece 1b, which is also formed by a plurality of bands with different elasticity; the bands of the front fabric piece 1a and the bands of the back fabric piece 1b, with the same elasticity, having at least one contact point;
2. Spraying a mixture consisting of resin and zeolite on the inside and/or outside of the garment;
3. Subsequently spraying a mixture consisting of resin and steatite on the inside and/or outside of the same garment;
4. Fixing the compound of resin and color through a wash at the dry cleaner's.
   In order to facilitate the lymphatic and venous circulation using the peculiarities of blood and lymph, which tend to move from the cold areas of the body to those warmer, the spraying of minerals is not uniform but more concentrated in certain areas of the garment.

## Claims

1. *A differentiated lymph drainage garment* **characterized by** at least two areas, a front one (1a) and a back one (1b) contiguous to each other, each made with a fabric divided into bands having a decreasing elasticity from the bottom upwards; the elasticity of the bands in the front area (1a) being, at the same height, different from or only partially coincident with the elasticity of the bands of the back area (1b).

2. A differentiated lymph drainage garment according to claim 1, wherein the fabric of the at least one front area (1a) consists of at least three bands with different elasticity, each of which is contiguous to one of the bands that make up the fabric forming the back area (1b), having the same elasticity degree.

3. A differentiated lymph drainage garment according to the preceding claims, wherein the fabric used for making the garment consists of a variable percentage of zeolite and steatite mineral.

4. A method for making a differentiated lymph drainage garment according to claim 1, **characterized by** the following steps:
- Tailoring the garment comprising a front fabric piece (la), formed by a plurality of bands with different elasticity, and a back fabric piece (1b), which is also formed by a plurality of bands with different elasticity; the bands of the front fabric piece (1a) and the bands of the back fabric piece (1b), with the same elasticity, having at least one contact point;
- Spraying a mixture consisting of resin and zeolite on the inside and/or outside of the garment;
- Subsequently spraying a mixture consisting of resin and steatite on the inside and/or outside of the same garment;
- Fixing the compound of resin and color through a wash at the dry cleaner's.

## Patentansprüche

1. *Differenziertes Lymphdrainagekleidungsstück,* das durch mindestens zwei Zonen, eine vordere (1a) und eine hintere (1b), die aneinander angrenzend angeordnet sind, hergestellt aus einem Stoff, der in Bändern unterteilt ist, die eine abnehmende Elastizität ab dem Hintern nach oben aufweisen; die Elastizität der Bänder der vorderen Zone (1a) ist auf gleicher Höhe, unterschiedlich oder nur teilweise mit der Elastizität der hinteren Zone (1b) übereinstimmend.

2. *Differenziertes Lymphdrainagekleidungsstück* nach Anspruch 1, worin der Stoff von mindestens einem vorderen Bereich (1a) aus mindestens drei Bändern mit unterschiedlicher Elastizität besteht, von denen jedes zu einem der Bänder, aus denen der Stoff besteht, der den hinteren Bereich (1b) bildet, konturiert ist, worin der Stoff den gleichen Elastizitätsgrad aufweist.

3. *Differenziertes Lymphdrainagekleidungsstück* nach vorhergehenden Ansprüchen, worin der zur Herstellung des Kleidungsstücks verwendeter Stoff aus einem variablen Prozentsatz von Zeolith und Steatitmineral besteht.

4. Die Methode für die Realisierung eines *differenzierten lymphdrainagekleidungsstücks* nach Anspruch 1, besteht aus den folgenden Phasen:
- Verpackung des Kleidungsstücks mit einem vorderen Stoffstück (1a), das aus einer Pluralität der Bänder mit unterschiedlicher Elastizität und einem hinteren Stoffstück (1b) besteht, das auch aus einer Pluralität der Bänder mit unterschiedlicher Elastizität besteht; die Bänder des vorderen Stoffstück (1a) und die Bänder des hinteren Gewebstuck (1b) haben die gleiche Elastizität und mindestens ein Gemeinschaftspunkt;
- Spritzer einer Mischung von Harz und Zeolith auf der Innenseite und/oder Außenseite des Kleidungsstücks;
- Nachfolgender Spritzer einer Mischung von Harz und Steatit auf der Innenseite und/oder Außenseite des Kleidungsstücks;
- Fixierung der Harzmasse und der Farbe durch Waschen in der Färberei.

## Revendications

1. *Vêtement de drainage lymphatique différencié* **caractérisé par** au moins deux zones, l'une antérieure (1a) et l'autre postérieure (1b), contiguës, chacune réalisée avec un tissu divisé en bandes élastiques exerçant une pression décroissante de bas en haut; l'élasticité des bandes de la zone antérieure (1a) étant, à la même hauteur, différente ou seulement partiellement coïncidente avec l'élasticité des bandes de la zone postérieure (1b).

2. *Vêtement de drainage lymphatique différencié* selon la revendication 1, dans lequel le tissu de la au moins une zone antérieure (la), est composé d'au moins trois bandes avec une élasticité différente, chacune d'elles étant contiguë à l'une des bandes composant le tissu formant la zone postérieure (1b), ayant le même degré d'élasticité.

3. *Vêtement de drainage lymphatique différencié* selon les revendications précédentes, dans lequel le tissu utilisé pour fabriquer le vêtement est constitué d'un pourcentage variable de minéral de zéolite et de stéatite.

4. *Méthode de réalisation d'un vêtement de drainage lymphatique différencié* selon la revendication 1, **caractérisé par** les phases suivantes:
- Confectionner le vêtement comprenant une pièce de tissu antérieure (la), formée par une pluralité de bandes avec élasticité différente, et une pièce de tissu postérieure (1b), également formée par une pluralité de bandes avec élasticité différente; les bandes de la pièce de tissu antérieure (1a) et les bandes de la pièce de tissu postérieure (1b), avec la même élasticité, ayant au moins un point de contact;
- Vaporiser, à l'intérieur et/ou à l'extérieur du vêtement, une mixture composée de résine et zéolite;
- Vaporiser ultérieurement, à l'intérieur et/ou à l'extérieur du même vêtement, une mixture composée de résine et stéatite;
- Fixer le composé de résine et de couleur par un lavage au pressing.
